# EUROPEAN PATENT APPLICATION

(11) **EP 0 767 376 A2**
(43) Date of publication of application: **09.04.1997**
(21) Application number: 96307270.7
(22) Date of filing: 04.10.1996
(51) Int. Cl.: G01N 33/543

(54) **Method for detecting or measuring an immunologically reactive substance**

(30) Priority: 06.10.1995 JP 284444/95
(71) Applicant: Karube, Isao, Kawasaki-shi, Kanagawa 216 (JP); TOKUYAMA CORPORATION, Tokuyama-shi, Yamaguchi-ken 745 (JP); A & T Corporation, Hino-shi, Tokyo 191 (JP)
(72) Inventor: Karube, Isao, Kawasaki-shi, Kanagawa-ken (JP); Iwata, Keisuke, Kuki-shi, Saitama-ken (JP); Yoshimura, Yoshimichi, Tsukuba-shi, Ibaraki-ken (JP)
(74) Representative: Woods, Geoffrey Corlett

(57) **Abstract**

A method for detecting or measuring an immunologically reactive substance through an immunological reaction with a reagent which is an antigen, an antibody, a carrier reagent sensitized by an antigen, or a carrier reagent sensitized by an antibody, wherein an immunological reaction product is produced in an aqueous reaction system by applying to the aqueous reaction system in which the reagent and the immunologically reactive substance are present a small voltage (preferably insufficient to cause electrolysis of the system) without substantially forming a pearl chain derived from the reagent, i.e. when the reagent includes a carrier three or more particles of carrier reagent are not arranged adjacent to one another in the form of a straight chain.

## Description

This invention relates to a method for detecting or measuring an immunologically reactive substance. More specifically, it relates to a method for carrying out the detection or measurement with high sensitivity and at high speed.

Since an immunologically reactive substance forms an insoluble agglomerate through an immunological agglutination reaction, the immunologically reactive substance can be detected or measured by detecting the insoluble aggregate formed. Heretofore, an enzyme immunoassay method or radio-immunoassay method has been widely used as a method for detecting or measuring an immunologically reactive substance, which makes use of this phenomenon. Although these methods feature high sensitivity and high accuracy, they have restrictions on the stability and storage of a reagent because they use radiation and an enzyme and require delicate consideration and high technique. Therefore, a simpler method has been desired.

In addition, since rapid examination is difficult with these methods because they need a considerably long measurement time, attempts have been made to research and develop a highly sensitive, rapid method.

Various optical analysis methods have been developed since 1970 as a method for measuring a latex agglutination reaction. The reaction temperature in these systems is 37-to 45°C and the reaction proceeds by stirring with stirring blades. The time required for measurement (reaction) at this point is about 10 to 20 min. Although this measurement time is shorter than that of the above enzyme immunoassay method or radio-immunoassay method, these optical analysis methods are inferior, though being improved stepwise, in measurement sensitivity and measurement range to these methods. Therefore, their application range is limited. In the medical field of today in which rapid examination is of great significance, the development of a much rapider examination method is desired.

As the measurement method making use of an immunological agglutination reaction, there has been known a method in which electric pulses are applied to a reaction system to accelerate such an immunological agglutination reaction. For instance, Tamiya et al. have reported that a suspension of antibodies for human immunoglobulin G which is bonded to latex particles in purified water was mixed with a solution of human immunoglobulin G in purified water in a cuvette equipped with electrodes, a pulse voltage having a pulse height of 200 V (field strength of 200 V/mm) was applied to the resulting mixture, and an agglutination ratio of 50 % was obtained 10 minutes later (refer to Biosensors, 3(3), pp. 139-146 (1988)).

However, since the promotion of an agglutination reaction is insufficient in this method in which electric pulses are applied, the method cannot fully meet demand for rapid and highly sensitive measurement. Further, this method has such a defect that electrolysis of a reaction solution is liable to occur. To prevent electrolysis, it is necessary to reduce the salt concentration of a reaction solution as much as possible. Therefore, the method in which electric pulses are applied requires pretreatment for adjusting the salt concentration of a reaction system in a measurement sample, particularly a sample of a living organism.

Iwata et al. have proposed a method for accelerating an immunological agglutination reaction and increasing the sensitivity of the reaction by applying an AC field of 5 to 50 V/mm for 30 to 60 seconds under the coexistence of 10 mM or more of salt (refer to JP-A 83928/1995 (the term "JP-A" as used herein means an "unexamined published Japanese patent application")). This method makes use of a pearl chain effect. However, it is difficult to form a pearl chain with latex particles having a particle diameter of 1 µm or less which are generally used and it is also difficult to implement this method with a commonly used optical detection system.

It is therefore an object of the present invention to provide a novel method for detecting or measuring an immunologically reactive substance.

It is another object of the present invention to provide a method which can detect or measure an immunologically reactive substance rapidly by promoting an immunological reaction extremely swiftly.

It is still another object of the present invention to provide a method for detecting or measuring an immunologically reactive substance, which can confirm the presence of an immunologically reactive substance with high accuracy.

The above and other objects and advantages of the present invention will become apparent from the following description.

According to the present invention, the above objects and advantages of the present invention can be attained by a method for detecting or measuring an immunologically reactive substance through an immunological reaction using a reagent selected from the group consisting of an antibody, an antigen, a carrier reagent sensitized by an antigen, and a carrier reagent sensitized by an antibody, wherein an immunological reaction product is produced in an aqueous reaction system by applying a small voltage to the aqueous reaction system in which the reagent and the immunologically reactive substance are present without substantially forming a pearl chain derived from the reagent.

### Brief Description of the Drawings

Fig. 1 is a schematic diagram of an apparatus suitable for use in the detection or measurement of an immunologically reactive substance, provided by the present invention;
Fig. 2 is a calibration curve obtained when a pulse voltage of 1.7 V/mm is applied to the reaction system of a myoglobin specimen;
Fig. 3 is a calibration curve obtained when a pulse voltage of 1.5 V/mm is applied to the reaction system of a myoglobin specimen;
Fig. 4 is a calibration curve obtained when a pulse voltage of 1.8 V/mm is applied to the reaction system of an AFP specimen;
Fig. 5 shows the relationship between the NaCl concentration and electroconductivity of an aqueous sodium chloride solution;
Fig. 6 shows the relationship between the electroconductivity and limited voltage of an aqueous sodium chloride solution;
Fig. 7 is a graph showing the relationship among human AFP concentration, AC voltage application and changes in turbidity;
Fig. 8 is a graph showing the relationship among human AFP concentration, AC voltage frequency and changes in turbidity;
Fig. 9 is a graph showing the relationship among human AFP concentration, AC voltage application time and changes in turbidity;
Fig. 10 is a graph showing the relationship between human AFP concentration and changes in turbidity depending on DC voltage application;
Fig. 11 is a graph showing the comparison between the calibration curves at a low concentration range of the prior art method and the method of the present invention in a turbidimetric immunoassay method; and
Fig. 12 is a graph showing the comparison between the calibration curves at a low concentration range of the prior art method and the method of the present invention in an turbidimetric immunoassay method.

The term "immunological reaction" as used in the present invention means an immunological agglutination reaction. The immunological agglutination reaction refers to a method making use of an antigen-antibody reaction, a reaction with a specific receptor or the like and includes both qualitative and quantitative methods.

The immunologically reactive substance in the present invention can be selected from substances which can be measured by an agglutination method using latex, liposome or the like as a carrier through the above immunological agglutination reaction. Illustrative examples of the immunologically reactive substance as a first group include infectious disease markers such as HBs antigen, HBs antibody, HCV antibody, HIV antibody and TP antibody; thrombosis and fibrinosis markers such as D-dimer, protein C, protein S and AT III; tumor markers such as MP, CEA and CA19-9; hormones such as TSH, prolactin and insulin; tissue elements such as β2m, myoglobin and myosin; and nucleic acid such as DNA. The detectable concentration of the immunologically reactive substance of the first group is about 0.05 ng/ml to 50 µg/ml, preferably 0.1 ng/ml to less than 50 µg/ml.

Illustrative examples of the immunologically reactive substance as a second group include serum proteins such as CRP, albumin, transferrin, myoglobin, LP(a), C4, C3, IgG, IgM, α1-antitrypsin and the like. The detectable concentration of the immunologically reactive substance of the second group is about 100 ng/ml to 1 mg/ml and the detection sensitivity is about 100 ng/ml to 10 µg/ml.

Illustrative examples of the reagent used in the present invention include an antigen, an antibody, a carrier reagent sensitized by an antigen and a carrier reagent sensitized by an antibody. They may be used alone or in combination of two or more.

Preferred examples of the carrier particle include latex, liposome, gold sol and the like. Latex particles are more preferred. Latex particles which are generally used by an immunological agglutination method can be used in the present invention. The average diameter of the carrier particles is preferably 0.01 to 1 µm, more preferably 0.05 to 0.5 µm, the most preferably 0.1 to 0.3 µm. When the average diameter is more than 1 µm, the Brovmian motion of the particles hardly occurs.

An immunological agglutination reaction is preferably carried out between the immunologically reactive substance of the first group and a carrier reagent sensitized by an antigen or a carrier reagent sensitized by an antibody as the reagent.

Preferably, the reagent is a carrier reagent sensitized by an antigen or a carrier reagent sensitized by an antibody, out of the above reagents, and the carrier itself is latex particles or liposome particles.

The sensitization of an antigen or antibody to particles can be carried out by adsorbing or bonding an antibody or antigen to particles by a conventionally known method.

An immunological agglutination reaction is preferably carried out between the immunologically reactive substance of the second group and an antigen or antibody as the reagent.

As the antigen or antibody used as the reagent, for example, an antibody which can immunologically identify the above serum proteins may be used.

The reagent used in the present invention is generally dispersed in an aqueous medium. Although the salt concentration of the aqueous medium containing the reagent is not particularly limited, it is such that electroconductivity preferably becomes 1 to 100 mS/cm, more preferably 2 to 60 mS/cm. Illustrative examples of the salt include potassium chloride, sodium chloride, sodium nitrate and ammonium nitrate, out of which potassium chloride and sodium chloride are particularly preferred. As the composition of the aqueous medium containing the reagent can be used one which is well known to one of ordinary skill in the art. For example, the aqueous medium may contain protein such as albumin, surfactant such as polyethylene glycol, Tween or triton, chloride such as choline chloride, amino acid such as arginine or asparagine, antiseptic such as sodium azide, and the like. The electroconductivity of the reaction system is preferably small because the field strength to be applied to the reaction system can be increased when the electroconductivity is small, thereby accelerating an immunological agglutination reaction advantageously.

In the method of the present invention, a small voltage is applied to the aqueous reaction system in which the reagent and the immunologically reactive substance are present.

The small voltage may be derived from pulses, DC or AC. When the voltage is derived from pulses, it is preferably in the range of 0.1 to 10 V/mm, more preferably 0.3 to 5 V/mm. The pulse width is preferably in the range of 1 to 100 µs, more preferably 10 to 50 µs. The frequency of the pulse is preferably in the range of 1 KHz to 1 MHz.

When the voltage is derived from DC, it is preferably in the range of 0.1 to 1 V/mm, more preferably 0.2 to 0.5 V/mm.

Further, when the voltage is derived from AC, the peak-to-peak voltage is preferably in the range of 0.1 to 20 V/mm, more preferably 0.5 to 10 V/mm. The frequency of AC is preferably in the range of 1 KHz to 1 MHz. Although AC voltage may be continuous, pulse or any wave in waveform, it is preferably sine wave, square wave or rectangular wave.

The small voltage derived from any one of the above is advantageously a voltage which does not substantially cause the electrolysis of the aqueous reaction system.

The voltage which does not substantially cause the electrolysis of the reaction system is, for example, a pulse voltage having a field strength of 2.4 V/mm (pulse width: 10 µs, frequency: 10 KHz) or an AC voltage having a field strength of 7.8 V/mm or less (frequency: 10 KHz) when the electroconductivity of the reaction system is 17.4 mS/cm (equivalent to that of physiologic saline). This voltage varies according to the material and area of the electrode, application time and the like.

In the method of the present invention, substantially no pearl chain derived from the reagent is formed in the aqueous reaction system by applying a small voltage to the reaction system. The term "pearl chain" as used herein means three or more particles of the carrier reagent arranged adjacent to one another in the form of a straight chain.

The method of the present invention can be embodied in various ways as follows depending on the application of a small voltage and timing with detection or measurement.
(1) A first method comprising the steps of applying a small voltage to an aqueous reaction system in which a reagent and an immunologically reactive substance are present, stopping the application and thereafter detecting or measuring the immunologically reactive substance;
(2) a second method comprising the steps of applying a small voltage to an aqueous reaction system in which a reagent and an immunologically reactive substance are present and detecting or measuring the immunologically reactive substance while continuing the application;
(3) a third method comprising the steps of applying a small voltage to an aqueous medium containing a reagent, adding an immunologically reactive substance while applying the voltage, stopping the application after the addition is completed and detecting or measuring the immunologically reactive substance; and
(4) a fourth method comprising the steps of applying a small voltage to an aqueous medium containing a reagent, adding an immunologically reactive substance while applying the voltage, and detecting or measuring the immunologically reactive substance while continuing the application after the addition is completed.

In any one of the above embodiments, the immunologically reactive substance is preferably pretreated in a buffer solution at room temperature to 37°C for 10 seconds to 10 minutes before it is mixed with the reagent. The buffer solution preferably contains a substance for eliminating a non-specific agglutination reaction and/or a sensitizing agent.

Illustrative examples of the substance for eliminating a non-specific agglutination reaction include dithiothreitol, 2-mercaptoethanol and urea. Illustrative examples of the sensitizing agent include polyethylene glycol and dextran.

In the method of the present invention, the immunologically reactive substance can be detected or measured by detecting or measuring an immunological reaction product in the reaction system by a latex photometric immunoassay method or counting immunoassay method, for example.

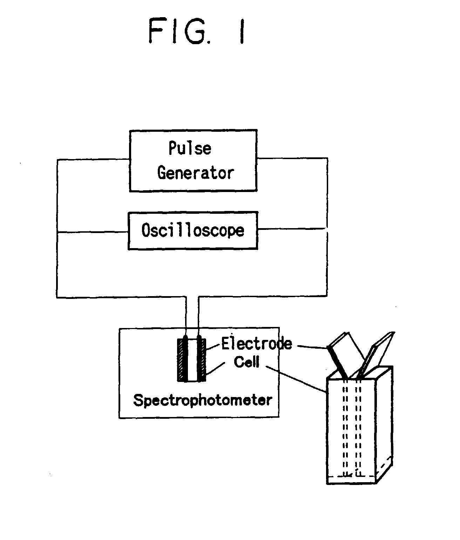

A typical apparatus for applying a small voltage to the reagent and a typical measuring instrument for detecting or measuring the immunologically reactive substance in the present invention comprise, as shown in Fig. 1, a power supply unit capable of applying any voltage such as a pulse voltage, AC voltage or DC voltage, a spectrophotometric cell with electrodes, an oscilloscope for monitoring voltage, frequency or pulse width, a spectrophotometer for measuring turbidity and the like, respectively. As the power supply unit may be used any pulse generator such as a commercial function generator or cell stimulation unit. As the spectrophotometric cell may be used a commercial glass cell or plastic cell, and as the electrode material may be used platinum or nickel.

To further illustrate the present invention, and not by way of limitation, the following Examples and Comparative Examples are given.

### Example 1

### (1) Preparation of reagent A sensitized by anti-myoglobin antibody

2.5 mg of an anti-myoglobin antibody was dissolved in 9.5 ml of a phosphate buffer solution (containing 20 mM phosphate, 50 mM sodium chloride and 0.05 % sodium azide, to be abbreviated as PBS hereinafter) and 0.5 ml of latex particles (particle diameter:0.22 µm, a suspension having a 10 % solid content, manufactured by Sekisui Chemical Co. Ltd.) was added to the solution and stirred at room temperature for 2 hours. A supernatant was removed by centrifuging the thus sensitized latex. The precipitate was suspended in 10 ml of a glycine buffer solution of 0.2 % bovine serum-albumin (0.1M glycine, 50 mM sodium chloride, 0.05 % sodium azide and 0.2 % BSA, to be abbreviated as 0.2 % BSA-GBS hereinafter) to prepare a latex reagent A sensitized by an anti-myoglobin antibody.

### (2) Calibration

### <Specimen>

A myoglobin positive control was diluted to predetermined concentrations (6400, 3200, 1600, 800, 400, 200, 100, 50, 25, 12.5, 6.24, 3.12, 1.56, 0.78 ng/ml) using 0.2 % BSA-GBS to prepare specimens.

### <Measuring Instrument>

A spectrophotometer was used and electrodes were installed on a glass cell having an optical path of 1 cm to measure at a wavelength of 800 nm.

### <Method>

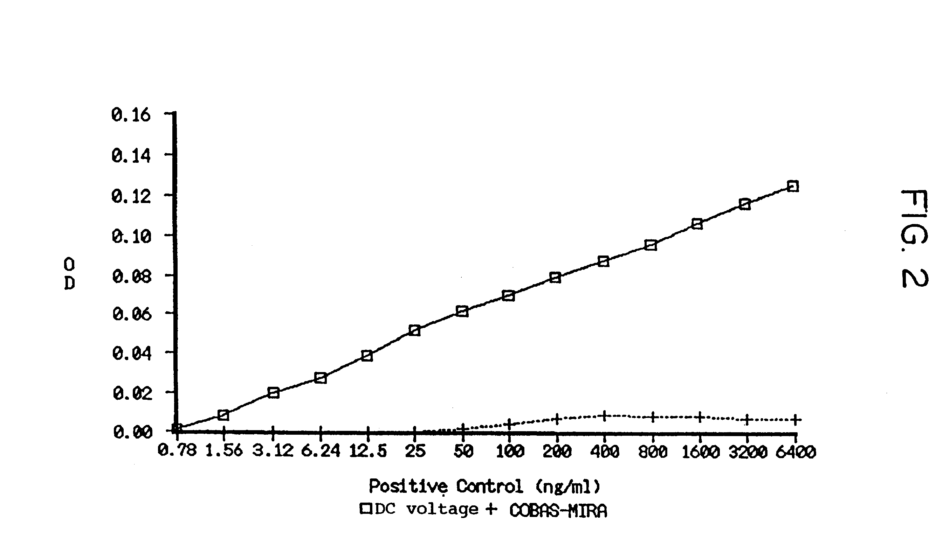

25 µl of each of the specimens was taken into the glass cell with electrodes and diluted by adding 455 µl of 0.2 % BSA-GBS. To this was added 20 µl of the latex reagent A sensitized by an anti-myoglobin antibody. Immediately after mixing, the resulting mixture was measured for its absorbance (turbidity) using a measuring instrument [Al]. A pulse voltage of 1.7 V/mm (pulse width: 15 µsec, frequency: 4 KHz) was applied to supply a small current for 5 seconds. Immediately after mixing, the mixture was measured for its absorbance (turbidity) using the measuring instrument [A2]. A change in absorbance (turbidity) [A2-A1] at each concentration was calculated and a calibration curve shown in Fig. 2 (□) was obtained. This calibration curve is represented by ΔOD/5 sec.

### Comparative Example 1

A calibration curve drawn by a fully automatic analyzer (COBAS-MIRA of Nippon Roche K.K.) using the latex reagent A sensitized by an anti-myoglobin antibody and the specimens used in Example 1 is shown in Fig. 2 (+).

The final concentration of this latex at the time of reaction was the same as in Example 1 and an immunological reaction was carried out at 37°C for 325 seconds and measured as a rate assay. This calibration curve is represented by ΔOD/ruin.

As is evident from Fig. 2, extremely short-time, high-sensitivity measurement at a wide range of concentration is possible with the method of the present invention, compared with the prior art method (a rate assay method having an incubation time of 5 to 20 min. at 37°C).

### Example 2

### (1) Preparation of reagent B sensitized by anti-myoglobin antibody

1.5 mg of an anti-myoglobin antibody was dissolved in 9.5 ml of PBS and 0.5 ml of latex having a particle diameter of 0.6 µm (a suspension having a 10 % solid content, manufactured by Sekisui Chemical Co. Ltd.) was added to the solution to prepare a latex reagent B sensitized by an anti-myoglobin antibody in the same manner as in Example 1.

### (2) Calibration

### <Specimen>

A myoglobin positive control was diluted to predetermined concentrations (1600, 800, 400, 200, 100, 50, 25 ng/ml) using 0.2 % BSA-GBS to prepare specimens.

### <Measuring Instrument>

The same measuring instrument as in Example 1 was used to measure at a wavelength of 850 nm.

### <Method>

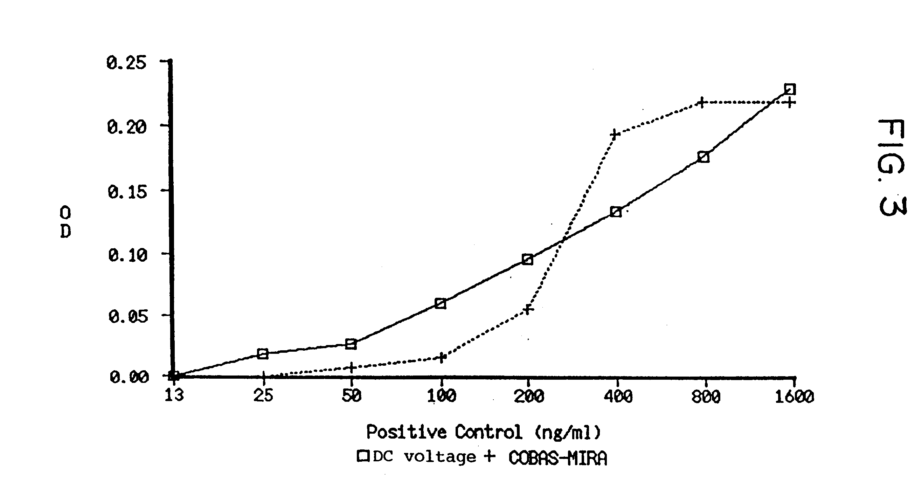

20 µl of each of the specimens was taken into the glass cell with electrodes and diluted by adding 460 µl of 0.2 % BSA-GBS. To this was added 20 µl of the latex reagent B sensitized by an anti-myoglobin antibody. Immediately after mixing, the resulting mixture was measured for its absorbance (turbidity) using a measuring instrument [A1]. A pulse voltage of 1.5 V/mm (pulse width: 15 µsec, frequency: 8 KHz) was applied to supply a small current for 10 seconds. Immediately after mixing, absorbance (turbidity) was measured using the measuring instrument [A2]. A change in absorbance (turbidity) [A2-A1] was calculated as in Example 1 and a calibration curve shown in Fig. 3 (□) was obtained. This calibration curve is represented by ΔOD/10 sec.

### Comparative Example 2

The operation of Example 2 was repeated using the specimens and the latex reagent B sensitized by an anti-myoglobin antibody of Example 2 to obtain A1. Immediately after 5 minutes of incubation at 37°C under this state, absorbance (turbidity) was measured [A2]. Similarly, a change in absorbance (turbidity) [A2-A1] was calculated and a calibration curve shown in Fig. 3 (+) was obtained. This calibration curve is represented by ΔOD/min.

As is evident from Fig. 3, according to the method of the present invention, a high-sensitivity reaction can be obtained at a low concentration range of a detected substance and a linear reaction can be obtained at a range from a medium concentration to a high concentration with a latex reagent using relatively large particles. Therefore, linearity can be obtained at a wide concentration range.

### Example 3

### (1) Preparation of latex reagent C sensitized by anti-AFP antibody

5 mg of an anti-AFP antibody was dissolved in 9.5 ml of a glycine buffer solution (to be abbreviated as GBS hereinafter) and 0.5 ml of latex particles (particle diameter: 0.2 µm, a suspension having a 10 % solid content) was added to the solution and stirred at room temperature for 2 hours. A supernatant was removed by centrifuging the sensitized latex suspension. The precipitate was suspended- in 100 ml of 0.2 % BSA-GBS to prepare an AFP latex reagent.

### (2) Calibration

### <Specimen>

An AFP positive control was diluted to predetermined concentrations (2.5, 1.25, 0.625, 0 ng/ml) using 0.2 % BSA-GBS to prepare specimens.

### <Measuring Instrument>

The same measuring instrument as in Example 1 was used to measure at a wavelength of 572 nm.

### <Method>

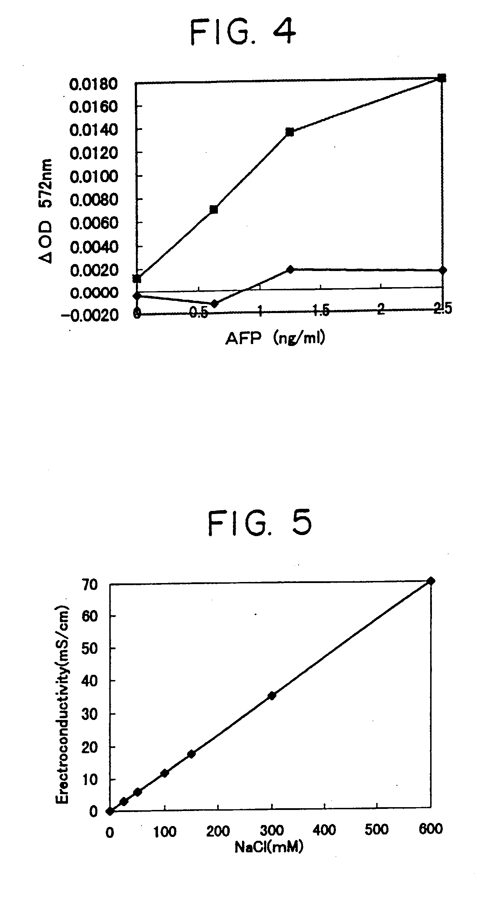

60 µl of each of the specimens was taken into the glass cell laminated with electrodes. To this were added 600 µl of a phosphate buffer solution (comprising 15 mM phosphoric acid, 0.4 % polyethylene glycol 6000, 2 % sodium chloride and 0.1 % sodium azide and containing 0.2 % bovine serum-albumin, to be abbreviated as 0.2 % BSA-PBS hereinafter) and 300 µl of the AFP latex reagent. Immediately after mixing, turbidity was measured using the measuring instrument [A1]. Thereafter, a DC pulse voltage of 1.8 V/mm (pulse width: 20 µsec, frequency: 10 KHz) was applied for 15 seconds. Immediately after mixing, turbidity was measured using the measuring instrument [A2]. A change in turbidity [A2-A1] was calculated as in Example 1. The results are shown in Table 1. A calibration curve shown in Fig. 4 (-■-) was obtained from the results of Table 1. This calibration curve is represented by ΔOD/15 sec.

### Comparative Example 3

The operation of Example 2 was repeated using the specimens and the latex reagent C sensitized by an anti-AFP antibody of Example 3 to measure A1. Immediately after 5 minutes of incubation at 37°C in this state, turbidity was measured [A2]. A change in turbidity for 5 minutes was obtained from [A2-A1]. The results are shown in Table 1. A calibration curve shown in Fig. 4 (-●-) was obtained from the results of Table 1.

**Table 1**

| AFP ng/ml | Comparative Example 3 Usual assay | Example 3 Pulse-assay |
|---|---|---|
| 0 | -0.0004 | 0.0012 |
| 0.625 | -0.0012 | 0.0070 |
| 1.25 | 0.0018 | 0.0135 |
| 2.5 | 0.0015 | 0.0180 |

As is apparent from Fig. 4, with the method of the present invention, it is possible to accelerate an immunological agglutination reaction and detect or measure the presence of an immunologically reactive substance with high sensitivity, compared with the method of the prior art (Comparative Example 3).

### Example 4 (measurement of voltage for inducing

### electrolysis)

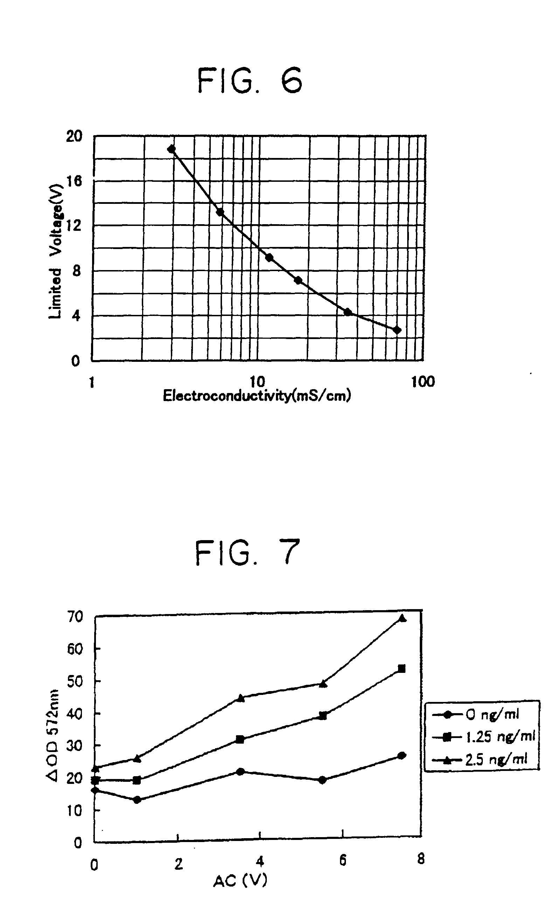

Sodium chloride was dissolved in purified water to sodium chloride concentrations of 0 to 600 mM. The electroconductivity and electrolysis starting voltage of the sodium chloride aqueous solution having each concentration were measured. A DC pulse voltage (pulse width: 10 µsec, frequency: 10 KHz) was applied. The results are shown in Table 2, Fig. 5 and Fig. 6.

**Table 2**

| Nacl (mM) | Electro-conductivity (mS/cm) | Limited Voltage (v) |
|---|---|---|
| 0 | 0 | 50 |
| 25 | 2.9 | 18.85 |
| 50 | 5.8 | 13.15 |
| 100 | 11.6 | 9.12 |
| 150 | 17.4 | 7.14 |
| 300 | 34.8 | 4.27 |
| 600 | 70 | 2.71 |

It is understood from Fig. 5 that electroconductivity and salt concentration are proportional to each other and from Fig. 6 that the voltage for inducing electrolysis is a logarithmic function of electroconductivity. That is, as the electroconductivity of the reagent decreases, it is less likely that electrolysis occurs.

### Example 6 (AC voltage characteristics/application voltage field strength)

### (1) Preparation of AFP latex reagent

5 mg of an anti-AFP antibody was dissolved in 9.5 ml of a glycine buffer solution (to be abbreviated as GBS hereinafter) and 0.5 ml of latex particles (particle diameter: 0.22 µm, a suspension having a 10 % solid content) was added to the solution and stirred at room temperature for 2 hours. The latex suspension thus sensitized was centrifuged to remove a supernatant. The precipitate was suspended in 33 ml of 0.2 % BSA-GBS to prepare an AFP latex reagent.

### (2) AC voltage application apparatus

The experimental apparatus of Fig. 1 in which the pulse generator was replaced by an AC voltage application apparatus was used and AC voltage was applied by setting the condition of a potential to be applied between electrodes by an oscilloscope (distance between electrodes: 3 mm).

### (3) Method

600 µl of 0.2 % BSA-PBS containing 0.2 % bovine serum-albumin and 300 µl of the AFP latex reagent were injected into a glass cell laminated with electrodes separately, and AC voltage (0 to ± 7.5 V, frequency: 10 KHz, sine wave) was applied for 2 minutes. Thirty seconds after the start of application, 60 µl of a specimen was added and a time course of change in turbidity was measured using a spectrophotometer (U-3200 of Hitachi) to obtain a turbidity difference during 5 minutes (ΔOD: 572 nm). Using 0.2 % BSA-GBS, an AFP reference was diluted to prepare specimens having AFP concentrations of 0 to 2.5 ng/ml and use them for measurement. The field strength of application voltage was set to 0 V, ±1 V, ±3.5 V, ±4.5 V and ±7.5 V to measure the specimens having respective concentrations.

### (4) Results

The results are shown in Table 3 and Fig. 7. As is apparent from Fig. 7, according to the present invention, it is possible to accelerate an immunological agglutination reaction and detect or measure an immunologically reactive substance with high sensitivity, compared with the method of the prior art (application voltage: 0 V). It is also understood that when the field strength of application voltage is increased, the immunological agglutination reaction can be further accelerated.

**Table 3**

| | ΔOD value (count value) at each AC application voltage | | | | |
|---|---|---|---|---|---|
| AFP (ng/ml) | 0 V | 1 V | 3.5 V | 5.5 V | 7.5 V |
| 0 | 16 | 13 | 21 | 18 | 25 |
| 1.25 | 19 | 19 | 31 | 38 | 52 |
| 2.5 | 23 | 26 | 44 | 48 | 68 |

### Example 7 (AC voltage characteristics/frequency)

### (1) Preparation of AFP latex reagent

An AFP latex reagent was prepared in the same manner as in Example 6.

### (2) AC voltage application apparatus

The same apparatus as in Example 6 was used for measurement.

### (3) Method

AC voltage (sine wave) having a frequency of 10 Hz to 100 KHz and a field strength of ±6 V was applied and a change in turbidity of each specimen by frequency was measured in the same manner as in Example 6.

### (4) Results

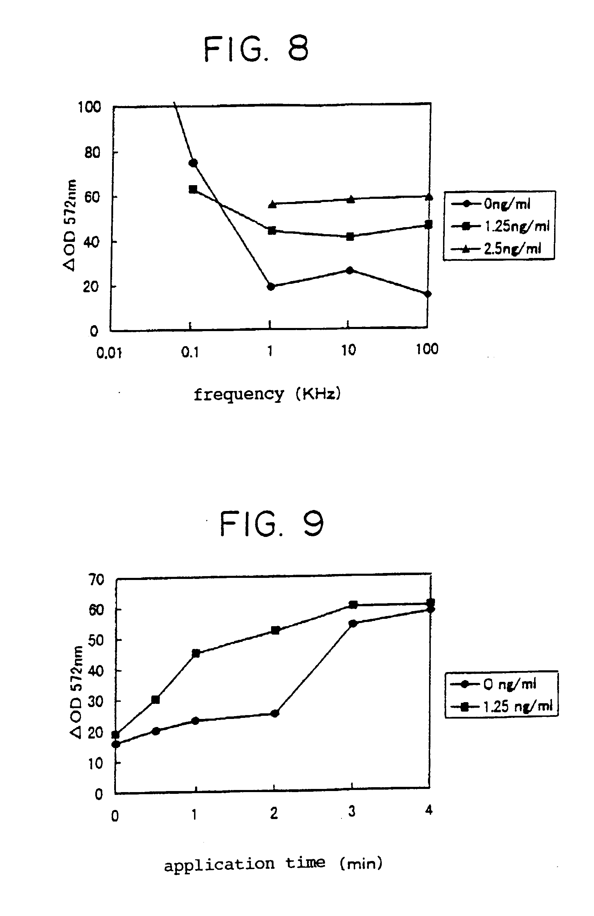

The results are shown in Table 4 and Fig. 8. It is understood from the results shown in Fig. 8 that an immunological agglutination reaction can be uniformly accelerated at an AC frequency of 1 to 100 KHz.

**Table 4**

| | ΔOD value (count value) at each frequency | | | | |
|---|---|---|---|---|---|
| AFP (ng/ml) | 0.01 KHz | 0.1 KHz | 1 KHz | 10 KHz | 100 KHz |
| 0 | 180 | 75 | 19 | 26 | 15 |
| 1.25 | - | 63 | 44 | 41 | 46 |
| 2.5 | - | - | 56 | 58 | 59 |

### Example 8 (AC voltage characteristics/application time)

### (1) Preparation of AFP latex reagent

An AFP latex reagent was prepared in the same manner as in Example 6.

### (2) AC voltage application apparatus

The same apparatus as in Example 6 was used for measurement.

### (3) Method

AC voltage (sine wave) having a frequency of 10 Hz and a field strength of ±7.5 V was applied for 0 to 4 minutes and a change in turbidity of each specimen by application time was measured in the same manner as in Example 6.

### (4) Results

The results are shown in Table 5 and Fig. 9. It is understood from the results shown in Fig. 9 that an immunological agglutination reaction can be accelerated when the application time of AC voltage is prolonged.

**Table 5**

| AFP | ΔOD value (count value) at each application time | | | | | |
|---|---|---|---|---|---|---|
| (ng/ml) | No appln. | 0.5 min. | 1 min. | 2 min. | 3 min. | 4 min. |
| 0 | 16 | 20 | 23 | 25 | 54 | 58 |
| 1.25 | 19 | 30 | 45 | 52 | 60 | 60 |
| appln.: application, min.: minutes | | | | | | |

### Example 9

### (1) Preparation of AFP latex reagent

An AFP latex reagent was prepared in the same manner as in Example 6.

### (2) DC voltage application apparatus

The experimental apparatus of Fig. 1 in which the pulse generator was replaced by a DC voltage application apparatus was used and DC voltage was applied by setting the condition of a potential to be applied between electrodes by an oscilloscope (distance between electrodes: 3 mm).

### (3) Method

600 µl of 0.2 % BSA-PBS and 300 µl of the AFP latex reagent were injected into a glass cell laminated with electrodes separately, and a DC voltage of 1 V was applied for 50 seconds. Ten seconds after the start of application, 60 µl of a specimen was added and turbidity was measured using a spectrophotometer (U-3200 of Hitachi) immediately. After five minutes, the mixture was stirred and turbidity was measured to obtain a turbidity difference during 5 minutes (ΔOD: 572 nm). Using 0.2 % BSA-GBS, an AFP standard was diluted to prepare specimens having AFP concentrations of 0 to 1.25 ng/ml and use them for measurement.

### (4) Results

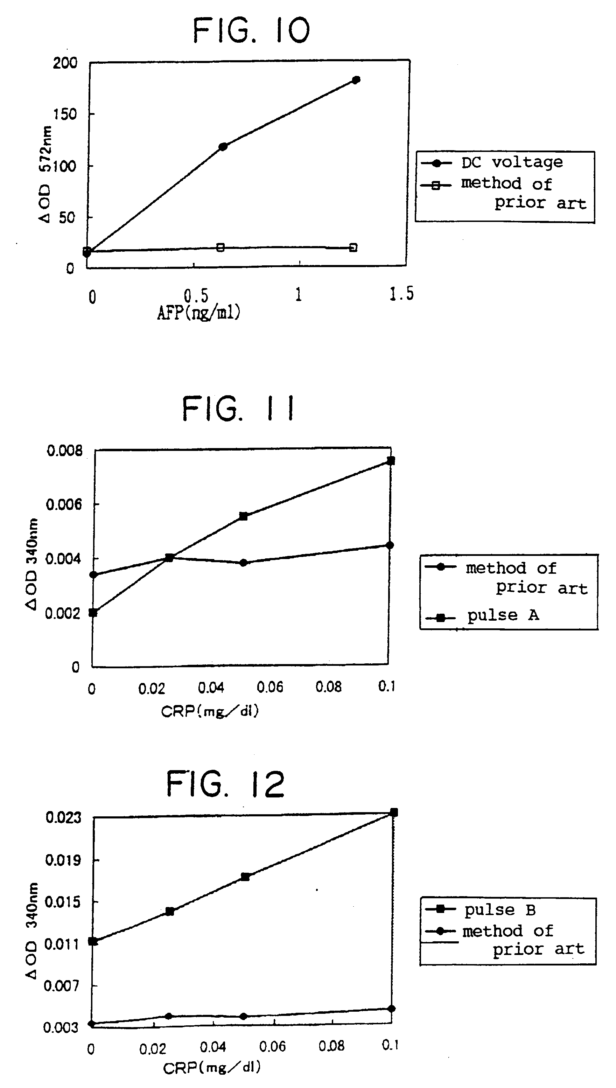

The results are shown in Table 6 and Fig. 10. As is evident from Fig. 10, according to the present invention, it is possible to accelerate an immunological agglutination reaction and detect or measure an immunologically reactive substance with high sensitivity, compared with the method of the prior art (application voltage: 0 V).

**Table 6**

| AFP | ΔOD value (count value) | |
|---|---|---|
| (ng/ml) | DC voltage application | Prior art method |
| 0 | 13 | 16 |
| 0.65 | 115 | 21 |
| 1.25 | 185 | 20 |

### Example 10

### (1) CRP reagent for latex photometric immunoassay

N-Assay TIA CRP-S (Nittobo Medical Co.) was used as a reagent for latex photometric immunoassay. The reagent consisted of a tris buffer solution and an antiserum solution (20 % anti-CRP caprine serum antibody).

### (2) DC pulse voltage application apparatus

The experimental apparatus of Fig. 1 was used and pulse voltage was applied by setting the condition of a potential to be applied between electrodes by an oscilloscope (distance between electrodes: 3 mm).

### (3) Measurement method

840 µl of a tris buffer solution and 36 µl of a specimen were injected into a glass cell laminated with electrodes separately. It was incubated at 37°C for 5 minutes and then, absorbance (ODI) was measured. Thereafter, 120 µl of an antiserum solution (20% anti-CRP caprine serum antibody) was added and DC pulse voltage (wave height value: 6 V, pulse width: 10 µs, frequency: 10 KHz) was applied for 30 seconds. It was incubated for another 5 minutes and tehn, absorbance (OD2) was measured again. The ΔOD value (OD2-OD1) was obtained for each specimen and a calibration curve was drawn. Using a phosphate buffer solution containing 0.2 % bovine serum-albumin (to be abbreviated as 0.2 % BSA-PBS hereinafter), a commercial CRP reference solution was diluted to prepare specimens having CRP concentrations of 0 to 4.1 mg/dl and use them for measurement.

### (4) Results

The results are shown in Table 7 and Fig. 11.

**Table 7**

| CRP mg/dl | Prior art method | Pulse A |
|---|---|---|
| 0 | 0.0034 | 0.0020 |
| 0.025 | 0.0040 | 0.0040 |
| 0.05 | 0.0038 | 0.0055 |
| 0.1 | 0.0044 | 0.0075 |
| 4.1 | 0.0530 | 0.0570 |

### Example 11

The same specimens and reagent as in Example 10 were used and the operation of Example 10 was repeated except that pulse voltage to be applied to the reaction system was set at a wave height value of 4.6 V, pulse width of 20 µs and frequency of 10 KHz and applied for 18 seconds to measure a change in turbidity and draw a calibration curve. The results are shown in Table 8 and Fig. 12.

**Table 8**

| CRP mg/dl | Prior art method | Pulse B |
|---|---|---|
| 0 | 0.0034 | 0.0112 |
| 0.025 | 0.0040 | 0.0140 |
| 0.05 | 0.0038 | 0.0172 |
| 0.1 | 0.0044 | 0.0230 |

### Comparative Example 4

The same specimens and reagent as in Example 10 were used. The operation of Example 10 was repeated except that pulse voltage was not applied to the reaction system to measure a change in turbidity and draw a calibration curve. The results are shown in Fig. 11 and Fig. 12.

It is understood from the results shown in Fig. 11 and Fig. 12 that according to the method of the present invention, it is possible to accelerate an immunological agglutination reaction and detect or measure an immunologically reactive substance with high sensitivity, compared with the method of the prior art.

As described on the foregoing pages, according to the method of the present invention, since an immunological agglutination reaction proceeds at a high reaction rate, an immunologically reactive substance can be easily measured with high sensitivity and at high speed, compared with the method of the prior art.

## Claims

1. A method for detecting or measuring an immunologically reactive substance through an immunological reaction with a reagent which is an antigen, an antibody, a carrier reagent sensitized by an antigen, or a carrier reagent sensitized by an antibody, wherein an immunological reaction product is produced in an aqueous reaction system by applying a small voltage to the aqueous reaction system in which the reagent and the immunologically reactive substance are present, without substantially forming a pearl chain derived from the reagent.

2. The method of claim 1, wherein the small voltage is derived from pulses, DC or AC.

3. The method of claim 1 or 2, wherein the small voltage is a voltage that does not substantially cause the electrolysis of the aqueous reaction system.

4. The method of any one of claims 1 to 3, wherein the small voltage is derived from pulses and produces a field strength of from 0.1 to 10 V/mm.

5. The method of claim 4, wherein the pulse width of the pulses is from 1 to 100 µs and the frequency of the pulses is from 1 KHz to 1MHz.

6. The method of any one of claims 1 to 3, wherein the small voltage is derived from DC and produces a field strength of from 0.1 to 1 V/mm.

7. The method of any one of claims 1 to 3, wherein the small voltage is derived from AC and produces a field strength of from 0.1 to 20 V/mm (peak-to-peak voltage).

8. The method of claim 7, wherein the frequency of AC is in the range of 1 KHz to 1 MHz.

9. The method of any one of the preceding claims, wherein the electroconductivity of the aqueous reaction system is in the range of 1 to 100 mS/cm.

10. The method of any one of the preceding claims, wherein the reagent is a carrier reagent sensitized by an antigen or a carrier reagent sensitized by an antibody and the carrier itself is latex particles or liposome particles.

11. The method of claim 10, wherein the average particle diameter of the carrier particles is 0.01 to 1 µm.

12. The method of any one of the preceding claims, wherein the small voltage is applied to the aqueous reaction system in which the reagent and the immunologically reactive substance are present and the immunologically reactive substance is detected or measured after the application is stopped.

13. The method of any one of claims 1 to 11, wherein the small voltage is applied to the aqueous reaction system in which the reagent and the immunologically reactive substance are present and the immunologically reactive substance is detected or measured while the application is continued.

14. The method of claim 12 or 13, wherein the immunologically reactive substance is pretreated in a buffer solution at a temperature of from room temperature to 37°C for 10 seconds to 10 minutes before it is mixed with the reagent.

15. The method of claim 14, wherein the buffer solution contains a substance for eliminating a non-specific agglutination reaction and/or a sensitizing agent.

16. The method of any one of claims 1 to 11, wherein the small voltage is applied to an aqueous medium containing the reagent, the immunologically reactive substance is added while the voltage is applied, the application is stopped after the addition is completed, and the immunologically reactive substance is detected or measured.

17. The method of any one of claims 1 to 11, wherein the small voltage is applied to an aqueous medium containing the reagent, the immunologically reactive substance is added while the voltage is applied, and the immunologically reactive substance is detected or measured while the application is continued after the addition is completed.

18. The method of claim 16 or 17, wherein the immunologically reactive substance is pretreated in a buffer solution at room temperature to 37°C for 10 seconds to 10 minutes.

19. The method of claim 18, wherein the buffer solution contains a substance for eliminating a non-specific agglutination reaction and/or a sensitizing agent.

20. The method of any one of the preceding claims, wherein the immunologically reactive substance is detected or measured by detecting an immunological reaction product in the reaction system by a latex photometric immunoassay method or a counting immunoassay method.
